# EUROPEAN PATENT SPECIFICATION

(11) **EP 2 358 299 B1**
(45) Date of publication and mention of the grant of the patent: **15.02.2017**
(21) Application number: 09796196.5
(22) Date of filing: 16.12.2009
(51) Int. Cl.: A61F 2/01

(54) **BODY LUMEN FILTER RETRIEVAL SYSTEM AND METHODS FOR FILTERING A BODY LUMEN**
KÖRPERLUMENFILTERRÜCKNAHMESYSTEM UND VERFAHREN ZUM FILTERN EINES KÖRPERLUMENS
SYSTÈME DE RÉCUPÉRATION D'UN FILTRE DANS UNE LUMIÈRE DE CORPS ET PROCÉDÉS POUR FILTRER UNE LUMIÈRE DE CORPS

(30) Priority: 17.12.2008 US 138386 P
(43) Date of publication of application: 24.08.2011
(73) Proprietor: Abbott Laboratories Vascular Enterprises Limited, Dublin 2 (IE)
(72) Inventor: SHRIVASTAVA, Sanjay, Irvine CA 92603 (US)
(74) Representative: Peters, Hajo
(86) International application number: PCT/US2009/068259
(87) International publication number: WO 2010/077949

(56) References cited:
- WO-A2-02/11626
- US-A1- 2006 149 295
- US-A1- 2008 269 868

## Description

### CROSS-REFERENCE TO RELATED APPLICATIONS

This Patent Application claims the benefit of and priority to U.S. Provisional Patent Application having Serial Number 61/138,386, filed on December 17, 2008.

### FIELD OF THE INVENTION

The present invention relates generally to medical devices. More particularly the present invention relates to body lumen filter retrieval systems and methods for filtering a body lumen.

### BACKGROUND OF THE INVENTION

Vein thrombosis is a medical condition wherein a blood clot, or thrombus, has formed inside a vein. Such a clot often develops in the calves, legs, or lower abdomen, but can also affect other veins in the body. The clot may partially or completely block blood flow, and may break off and travel through the bloodstream. Commonly, the clot is caused by a pooling of blood in the vein, often when an individual is bed-ridden for an abnormally long duration of time, for example, when resting following surgery or suffering from a debilitating illness, such as a heart attack or traumatic injury. However, there are many other situations that cause the formation of a blood clot.

Vein thrombosis is a serious problem because of the danger that the clot may break off and travel through the bloodstream to the lungs, causing a pulmonary embolism. This is similar to a blockage of the blood supply to the lungs that causes severe hypoxia and cardiac failure, and frequently results in death. For many patients, anti-coagulant drug therapies may be sufficient to dissipate the clots. For example, patients may be treated with anticoagulants such as heparin and with thrombolytic agents such as streptokinase.

Unfortunately, some patients may not respond to such drug therapy or may not tolerate such therapy. Also, there may be other reasons why an anticoagulant is not desirable. For example, patients may have an acute sensitivity to heparin or may suffer from prolonged internal and/or external bleeding as a result of such drug therapies. Also, such drug therapies simply may be ineffective in preventing recurrent pulmonary emboli. In such circumstances, surgical procedures are required to reduce the occurrence of pulmonary emboli. Mechanical interruption of the inferior vena cava typically presents an effective method of preventing of pulmonary embolisms.

US 2006/0149295 A1 discloses a filter retrieval system comprising a catheter having a proximal end and a distal end and defining a lumen, said catheter including an aperture; and an elongate retrieval member slidably disposable within said lumen, said elongate retrieval member including a retrieval structure adapted to releasably engage at least a portion of a filter, said retrieval structure extending out from said lumen through said aperture.

### BRIEF SUMMARY

Other aspects and features of the present invention will become apparent from consideration of the following description in conjunction with the accompanying drawings.

An object of the present invention is to provide an improved filter retrieval system.

This object is solved with a filter retrieval system according to claim1. With such a filter retrieval system having a capture structure operatively associated with the delivery catheter, the retrieved filter is captured by the capture structure such that the lumen of the catheter is available for other purposes, such as providing a lumen for introducing a guide wire.

In another example, a filter retrieval system includes filter retrieval means including means for enclosing at least a portion of a filter in a collapsed state. The filter retrieval system includes means for engaging the filter at a deployment site. The means for engaging are configured to direct the filter into at least a portion of the filter retrieval means. The filter retrieval system includes means for delivering the filter retrieval means to the deployment site in a body lumen. The means for delivering has a proximal end and a distal end. The means for engaging is disposed near the distal end of the means for delivering.

In a further example, a method for retrieving a filter is disclosed. A filter retrieval system is advanced from an access site into a body lumen until a distal tip of the filter retrieval system approaches a deployment site. A retrieval structure is advanced towards a distal end of the filter retrieval system until the retrieval structure engages a filter. At least a portion of the filter is directed towards a distal end of the capture structure. The filter retrieval system with the filter is removed from the body lumen.

### BRIEF DESCRIPTION OF THE DRAWINGS

In order to describe the manner in which the above-recited and other advantages and features of the invention can be obtained, a more particular description of the invention briefly described above will be rendered by reference to specific embodiments thereof which are illustrated in the appended drawings. Understanding that these drawings depict only typical embodiments of the invention and are not therefore to be considered to be limiting of its scope, the invention will be described and explained with additional specificity and detail through the use of the accompanying drawings.
FIG. 1 illustrates a filter retrieval system in accordance with the various embodiments of the present invention.
FIG. 2 illustrates an exemplary subject for a body lumen filter.
FIG. 3A illustrates the filter retrieval system shown in FIG. 1 being delivered to a delivery site of an embodiment of an implantable lumen filter.

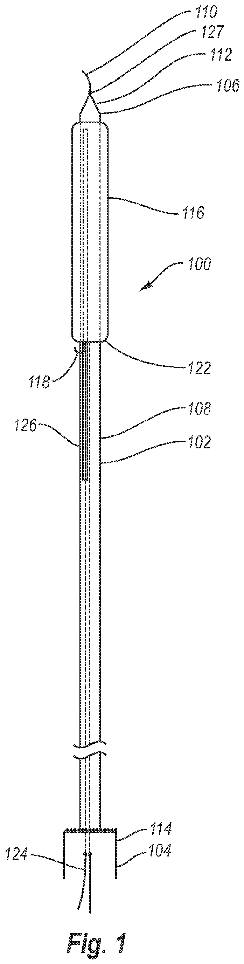

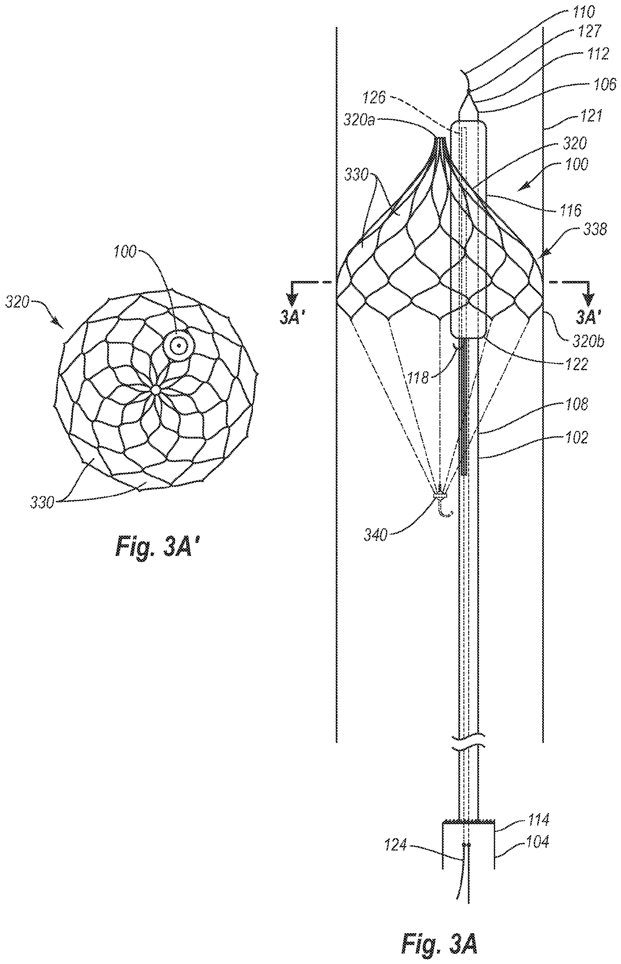

FIG. 3A' illustrates a top view of the filter retrieval system shown in FIG. 1 being delivered to a delivery site of an embodiment of an implantable lumen filter.

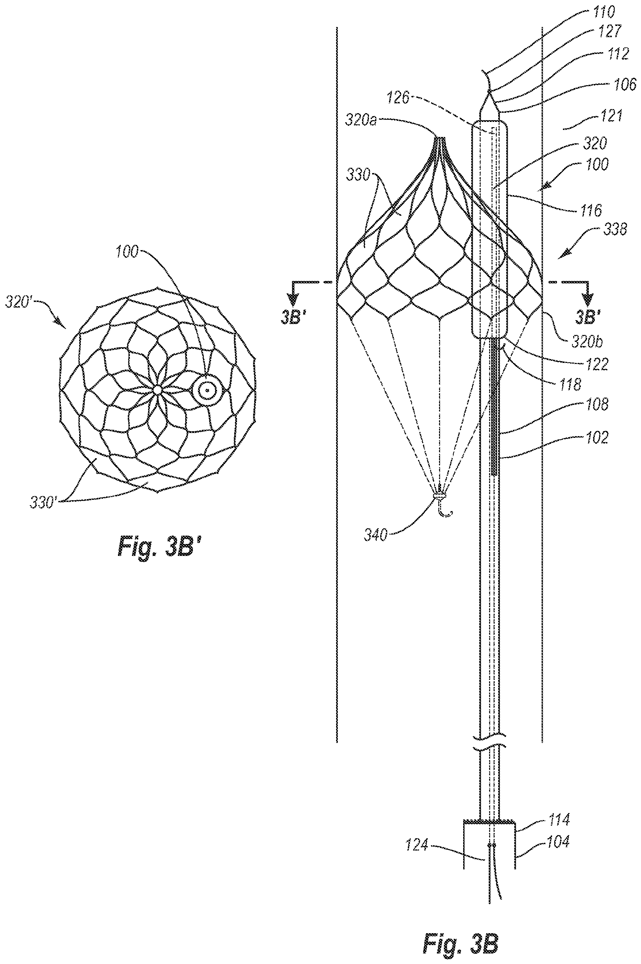

FIG. 3B illustrates the filter retrieval system shown in FIG. 1 being delivered to a delivery site of another embodiment of an implantable lumen filter.
FIG. 3B' illustrates a top view of the filter retrieval system shown in FIG. 1 being delivered to a delivery site of another embodiment of an implantable lumen filter.

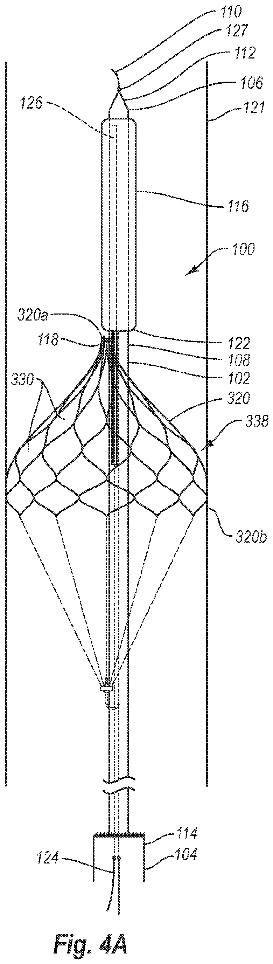

FIG. 4A illustrates the filter retrieval system shown in FIG. 1 engaging an embodiment of an implantable lumen filter.

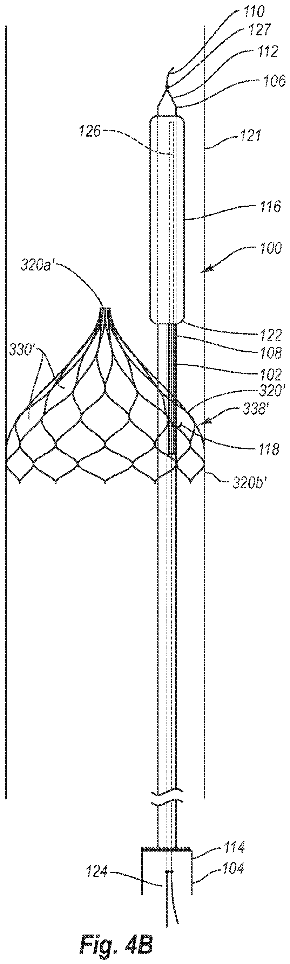

FIG. 4B illustrates the filter retrieval system shown in FIG. 1 engaging another embodiment of an implantable lumen filter.

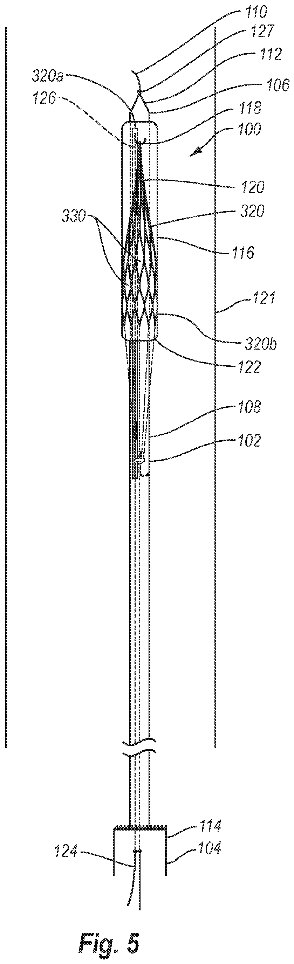

FIG. 5 illustrates directing the embodiment of an implantable lumen filter shown in FIG. 3A into the filter retrieval system shown in FIG. 1.

It should be noted that the figures are not drawn to scale and that elements of similar structures or functions are generally represented by like reference numerals for illustrative purposes throughout the figures. It also should be noted that the figures are only intended to facilitate the description of embodiments of the present invention.

### DETAILED DESCRIPTION

The various embodiments described herein extend generally to filter retrieval systems. By way of example only, implantable lumen filters, such as vena cava filters and other devices may be used to filter blood in blood vessels, which are discussed in reference to the vena cava filter retrieval systems. The various components of these filters and filter systems are also described. These components may include combinations of engagement portions, retrieval portions, and other components related to the vena cava filter retrieval system.

Some implantable lumen filters may be designed to capture and/or lyse particles of a particular size. An implantable lumen filter, when placed in a patient may prevent emboli of a particular size from reaching the pulmonary artery. Some implantable lumen filters may be placed in the inferior vena cava.

Some implantable lumen filters typically use jugular, antecubital, or other access sites for retrieval because they are typically not configured to be retrieved through the femoral access. Rather, some filters may include a distal retrieval structure, such as a hook.

Retrieval through the same access site through which the filter was deployed may be desired. For example, deployment and retrieval through a femoral access site may be desired. At least one embodiment of a filter retrieval system described herein allows for retrieval of conventional filters through the same or other access site through which the filter was deployed.

Embodiments of a filter retrieval system includes a capture structure configured to enclose at least a portion of a filter. The capture structure is located near the distal end of the filter retrieval system and defines an inner space by an at least partially closed distal end and an open proximal end. In some embodiments, the distal end may be completely closed. At least a portion of the filter is directed into the capture structure by an elongate retrieval member. The elongate retrieval member engages the filter and/or positions at least a portion of the filter within the capture structure using a retrieval structure, such as a hook. Some filters include a proximal and/or a distal retrieval structure, such as a hook, that may be engaged by the retrieval structure. Other filters may not include a retrieval structure, but may be engaged by the retrieval structure.

Referring now to the drawings, FIG. 1 illustrates a filter retrieval system 100 for a body lumen filter. The filter retrieval system 100 includes a delivery catheter 102 having a proximal end 104 and a distal end 106. A capture structure 116 is operatively associated with the delivery catheter 102 near the distal end of 106. The capture structure 116 includes an inner space defined by a distal end 116a and a proximal end 116b. The distal end 116a is at least partially closed and the proximal end 116b is at least partially open. The capture structure 116 is sized and configured to enclose at least a portion of a filter, such as filter 120 shown in FIG. 1, in a collapsed state. For example, the capture structure 116 is sized and configured to enclose an entire filter, such as filter 120 shown in FIG. 1.

The filter retrieval system 100 may include a retrieval component, the use of an embodiment of which is illustrated in FIGS. 3A-5. The retrieval component includes an elongate retrieval member 124 and a retrieval structure 118. The retrieval component is used to engage at least a portion of a filter and position at least a portion of the filter within the capture structure 116.

The filter retrieval systems described herein may be manufactured from any suitable material. For example, portions of a filter retrieval system may be, at least partially, formed from various materials such as biocompatible materials. Examples of such biocompatible materials for the filter retrieval systems can include a suitable hydrogel, hydrophilic polymer, biodegradable polymers, bioabsorbable polymers and bioneutral polymers. Examples of such polymers can include poly(alpha-hydroxy esters), polylactic acids, polylactides, poly-L-lactide, poly-DL-lactide, poly-L-lactide-co-DL-lactide, polyglycolic acids, polyglycolide, polylactic-co-glycolic acids, polyglycolide-co-lactide, polyglycolide-co-DL-lactide, polyglycolide-co-L-lactide, polyanhydrides, polyanhydride-co-imides, polyesters, polyorthoesters, polycaprolactones, polyesters, polyanydrides, polyphosphazenes, polyester amides, polyester urethanes, polycarbonates, polytrimethylene carbonates, polyglycolide-co-trimethylene carbonates, poly(PBA-carbonates), polyfumarates, polypropylene fumarate, poly(p-dioxanone), polyhydroxyalkanoates, polyamino acids, poly-L-tyrosines, poly(beta-hydroxybutyrate), polyhydroxybutyrate-hydroxyvaleric acids, other comparable materials, or combinations thereof. Other materials may include shape memory alloys, such as alloys of nickel titanium (nitinol). These materials may include at least one beneficial agent incorporated into the material and/or coated over at least a portion of the material.

The beneficial agents may be applied to filter retrieval systems that have been coated with a polymeric compound. Incorporation of the compound or drug into the polymeric coating of the filter retrieval systems can be carried out by dipping the polymer-coated implantable lumen filter into a solution containing the compound or drug for a sufficient period of time (such as, for example, five minutes) and then drying the coated implantable lumen filter, preferably by means of air drying for a sufficient period of time (such as, for example, 30 minutes). The polymer-coated filter retrieval systems containing the beneficial agent may then be used to retrieve an implantable lumen filter from a body vessel.

The beneficial agents that can be effective in preventing restenosis can be classified into the categories of anti-proliferative agents, anti-platelet agents, anti-inflammatory agents, anti-thrombotic agents, and thrombolytic agents. Anti-proliferative agents may include, for example, crystalline rapamycin. These classes can be further subdivided. For example, anti-proliferative agents can be anti-mitotic. Anti-mitotic agents inhibit or affect cell division, whereby processes normally involved in cell division do not take place. One sub-class of anti-mitotic agents includes vinca alkaloids. Representative examples of vinca alkaloids include, but are not limited to, vincristine, paclitaxel, etoposide, nocodazole, indirubin, and anthracycline derivatives, such as, for example, daunorubicin, daunomycin, and plicamycin. Other sub-classes of anti-mitotic agents include anti-mitotic alkylating agents, such as, for example, tauromustine, bofumustine, and fotemustine, and anti-mitotic metabolites, such as, for example, methotrexate, fluorouracil, 5-bromodeoxyuridine, 6-azacytidine, and cytarabine. Anti-mitotic alkylating agents affect cell division by covalently modifying DNA, RNA, or proteins, thereby inhibiting DNA replication, RNA transcription, RNA translation, protein synthesis, or combinations of the foregoing.

Anti-platelet agents are therapeutic entities that act by (1) inhibiting adhesion of platelets to a surface, typically a thrombogenic surface, (2) inhibiting aggregation of platelets, (3) inhibiting activation of platelets, or (4) combinations of the foregoing. Activation of platelets is a process whereby platelets are converted from a quiescent, resting state to one in which platelets undergo a number of morphologic changes induced by contact with a thrombogenic surface. These changes include changes in the shape of the platelets, accompanied by the formation of pseudopods, binding to membrane receptors, and secretion of small molecules and proteins, such as, for example, ADP and platelet factor 4. Anti-platelet agents that act as inhibitors of adhesion of platelets include, but are not limited to, eptifibatide, tirofiban, RGD (Arg-Gly-Asp)-based peptides that inhibit binding to gpIIbIIIa or αvβ3, antibodies that block binding to gpIIaIIIb or αvβ3, anti-P-selectin antibodies, anti-E-selectin antibodies, compounds that block P-selectin or E-selectin binding to their respective ligands, saratin, and anti-von Willebrand factor antibodies. Agents that inhibit ADP-mediated platelet aggregation include, but are not limited to, disagregin and cilostazol.

Anti-inflammatory agents can also be used. Examples of these include, but are not limited to, prednisone, dexamethasone, hydrocortisone, estradiol, fluticasone, clobetasol, and non-steroidal anti-inflammatories, such as, for example, acetaminophen, ibuprofen, naproxen, and sulindac. Other examples of these agents include those that inhibit binding of cytokines or chemokines to the cognate receptors to inhibit pro-inflammatory signals transduced by the cytokines or the chemokines. Representative examples of these agents include, but are not limited to, anti-IL1, anti-IL2, anti-IL3, anti-IL4, anti-IL8, anti-IL15, anti-IL18, anti-GM-CSF, and anti-TNF antibodies.

Anti-thrombotic agents include chemical and biological entities that can intervene at any stage in the coagulation pathway. Examples of specific entities include, but are not limited to, small molecules that inhibit the activity of factor Xa. In addition, heparinoid-type agents that can inhibit both FXa and thrombin, either directly or indirectly, such as, for example, heparin, heparin sulfate, low molecular weight heparins, such as, for example, the compound having the trademark Clivarin®, and synthetic oligosaccharides, such as, for example, the compound having the trademark Arixtra®. Also included are direct thrombin inhibitors, such as, for example, melagatran, ximelagatran, argatroban, inogatran, and peptidomimetics of binding site of the Phe-Pro-Arg fibrinogen substrate for thrombin. Another class of anti-thrombotic agents that can be delivered is factor VII/VIIa inhibitors, such as, for example, anti-factor VII/VIIa antibodies, rNAPc2, and tissue factor pathway inhibitor (TFPI).

Thrombolytic agents, which may be defined as agents that help degrade thrombi (clots), can also be used as adjunctive agents, because the action of lysing a clot helps to disperse platelets trapped within the fibrin matrix of a thrombus. Representative examples of thrombolytic agents include, but are not limited to, urokinase or recombinant urokinase, pro-urokinase or recombinant pro-urokinase, tissue plasminogen activator or its recombinant form, and streptokinase.

One or more immunosuppressant agents may be used. Immunosuppressant agents may include, but are not limited to, IMURAN® azathioprine sodium, brequinar sodium, SPANIDIN® gusperimus trihydrochloride (also known as deoxyspergualin), mizoribine (also known as bredinin), CELLCEPT® mycophenolate mofetil, NEORAL® Cylosporin A (also marketed as different formulation of Cyclosporin A under the trademark SANDIMMUNE®), PROGRAF® tacrolimus (also known as FK-506), sirolimus and RAPAMUNE®, leflunomide (also known as HWA-486), glucocorticoids, such as prednisolone and its derivatives, antibody therapies such as orthoclone (OKT3) and Zenapax®, and antithymyocyte globulins, such as thymoglobulins. In addition, a crystalline rapamycin analog, A-94507, SDZ RAD (a.k.a. Everolimus), and/or other immunosuppressants.

The capture structure 116 may be configured to receive at least a portion of a filter with the filter in a pre-deployed state (i.e. collapsed, longitudinally elongated, etc.). The capture structure 116 may be formed from a flexible material, such as silicone rubber, polyurethane, other flexible materials, or combinations thereof. The material can be similar to the material used in a balloon of a balloon catheter.

As mentioned above, the capture structure 116 may be expandable from a pre-capture state toward a capture state. The capture structure 116 is shown in FIG. 1 in a pre-capture state. The capture structure 116 may transition from the pre-capture state toward the capture state as at least a portion of a filter enters the capture structure 116. For instance, the material forming the capture structure 116 can be sufficiently flexible or elastic to, in one configuration, at least partially enlarge in diameter, while being sufficiently resilient to move the captured filter from an expanded configuration to a retrieved configuration within the capture structure 116. The capture structure 116 can then surround the captured filter and limit movement of the filter from within the capture structure 116. Optionally, the capture structure 116 can be formed from a flexible material with an elastic member disposed at a proximal end of the capture structure 116, this elastic member aiding with moving at least a portion of the capture structure 116 to the capture state with the filter in the captured state. In another configuration, the capture structure 116 can include a frame (not shown) supporting a flexible material, and/or the elastic member, the frame having sufficient flexibility to accommodate the captured filter, while sufficient resiliency to apply a force upon the filter to close and retrieve the filter, i.e., place the filter in the captured state.

In still another configuration, the capture structure 116 can be mechanically opened and closed using a tether or actuator that extends from the proximal end 104 toward the distal end 106 of the delivery catheter 102. The tether or actuator can extend to one or more frame members pivotally coupled to the distal end of the capture structure. These frame members can be biased to either the pre-capture state or the capture state, such as through the frame members being formed of a shape memory alloy (SMA), such as but not limited to Nitinol, or through the inclusion of a biasing member coupled to the frame members. When the frame members are biased to the pre-capture state, movement of the actuator in either the proximal or distal direction, depending upon the particular configuration, can release the frame members to move outwardly. Upon capture of the filter, movement in the direction opposite to that used to allow outward movement of the frame members allows the biasing frame members to return to the pre-capture state. It will be understood that when the frame members are biased open, movement of the tether or actuator would be used to enable the biased outward movement and subsequent closing of the capture structure.

In still another configuration, the capture structure 116 can include at least one balloon chamber (not shown) that may be inflated to expand the capture structure and/or apply a capture force to a filter received within the capture structure 116 to prevent movement of the filter following retrieval. The one or more balloon chambers may be formed over all or at least a portion of the capture structure, with these balloon chambers optionally being selectively inflatable during the capturing process. In embodiments where the capture structure 116 is inflated, one or more lumens in the delivery catheter 102 may also be provided and fluidly communicate with the one or more balloon chambers. The inclusion of the balloon can optionally be combined with the other configurations described herein, such as the biased frame members, etc.

In use, the capture structure 116 is disposed at and/or near the distal end 106 of the delivery catheter 102. The capture structure 116 may include a tapered proximal portion 122 which may taper toward the proximal end 116b.

The retrieval structure 118 is operatively associated with the retrieval member 124, which may be movable. The retrieval member 124 may be configured to facilitate movement of the retrieval structure 118 along the filter retrieval system 100 between the proximal and distal ends 104, 106 of the filter retrieval system 100. For instance, the retrieval member 124 may be a wire attached to the retrieval structure 118 and extending out from the handle 114 to be controlled by the user at and/or near the proximal end 104.

In the present embodiment, the delivery catheter 102 defines a lumen 108 and may be used in combination with a guide wire 110. In other embodiments, the filter retrieval system 100 may be otherwise guided toward a filter. For instance, an imaging device, such as a fluoroscope, x-ray, and/or other imaging device can be used to guide the filter retrieval system toward the filter.

The guide wire 110 can extend through the lumen 108 and extend from the lumen 108 through a distal tip 112 at and/or near the distal end 106. The lumen 108, for example, can have an inner diameter of about 1F to about 5F. The guide wire 110 can extend through the delivery catheter 102 from the proximal end 104 toward the distal end 106. The guide wire 110 may be accessible by a handle 114 at and/or near the proximal end 104 of the delivery catheter 102. The handle 114 may be configured to be grasped by a technician during insertion and/or removal of the delivery catheter 102.

The guide wire 110 may be used to guide a distal end 106 of the delivery catheter 102 toward the delivery site. The filter 120 may be disposed within the delivery catheter 102 in a collapsed state. While in the collapsed state, the filter 120 may be longitudinally elongated with respect to a deployed state.

The guide wire 110 may be removed after the distal end 106 of the delivery catheter 102 is located near the delivery site. Alternatively, the guide wire 110 may remain in the delivery catheter 102 during the filter retrieval process discussed above.

The retrieval structure 118 extends out from the lumen 108 through an aperture 126, such as an elongated slot, slit, or groove, disposed along an outer wall of the delivery system at and/or near the distal end 106. The aperture 126 extends from near the distal end 116a beyond the proximal end 116b of the capture structure 116.

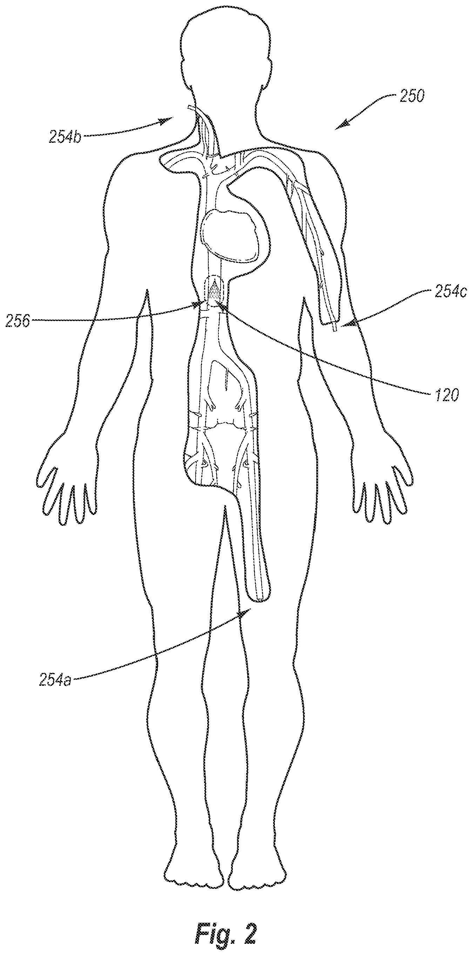

FIG. 2 illustrates an exemplary subject 250 for a body lumen filter 120. Although many of the embodiments herein may describe a body lumen filter 120, other filters may be deployed and/or retrieved using at least one embodiment of a filter retrieval system described herein. The filter 120 may be implanted in a body lumen 252 of the subject 250. The filter 120 may be inserted and/or retrieved through an access site 254a, 254b, 254c. In the present embodiment, the access site may include a femoral artery access site 254a, a jugular vein access site 254b, a radial vein access site 254c, femoral vein, brachial vein, brachial artery, other access sites, or combinations thereof. For instance, the filter 120 may be inserted through the femoral artery access site 254a and retrieved through the jugular or radial vein access site 254b, 254c. In another example, the filter 120 may be inserted through the jugular vein access site 254b and retrieved through the femoral artery or radial vein access site 254a, 254c. In a further example, the filter 120 may be inserted through the radial vein access site 254c and retrieved through the femoral artery or jugular vein access site 254a, 254b.

The filter 120 may be inserted and retrieved through the radial vein access site 254c. Additionally, the filter 120 may be inserted and retrieved through the jugular vein access site 254b. Further, the filter 120 may be inserted and retrieved through the femoral artery access site 254a.

The filter 120 may be deployed near a deployment site 256. In the present embodiment, the deployment site 256 may include a location within the inferior vena cava. In other embodiments, other deployment sites may be used, such as the superior vena cava. For example, the deployment site 256 may include all larger veins.

As mentioned above, some body lumen filters typically use jugular, antecubital, or other access sites for retrieval because they are typically not configured to be retrieved through the femoral access. Retrieval through the same access site through which the filter was deployed may be desired. At least in one embodiment of a filter retrieval system may provide for retrieval through the same access site through which the filter was deployed.

FIG. 3A illustrates the filter retrieval system 100 shown in FIG. 1 being delivered to a delivery site (shown as 256 in FIG. 2) of an embodiment of a filter 320. The filter 320 includes a body 338, such as a membrane, mesh, braded wire or frame, other body structures, or combinations thereof. The filter 320 may be disposed in a generally conical arrangement. A plurality of apertures 330 may extend through the thickness of the body 338.

Components of the filter retrieval system 100 may be sized and configured to position the proximal end 116b of the capture structure 116 beyond a distal end 320a of the filter 320 in a pre-capture state. For instance, the filter retrieval system 100 may be sized and configured to be inserted through at least one aperture 330. As illustrated in FIGS. 3A and 3A', the filter retrieval system 100 is inserted in a pre-capture state through an aperture 330 near the center of the body lumen 121 and/or filter 320. In other embodiments, the filter retrieval system 100 may be inserted through any appropriate aperture 330 or may be otherwise positioned.

The filter 320 may be configured to travel between a deployed (i.e. pre-capture) state and a pre-deployed (i.e. captured) state. The interior space of the capture structure 116 may be configured to be about the same or less than the general size of the body 338 in the pre-deployed state. The deployed diameter of the body 338 may allow the filter 320 to expand to a diameter that substantially traverses the cross sectional area of the body lumen being treated.

The filter 320 can be made from similar materials and/or components of the filter retrieval system 100 or other materials and/or coatings. Apertures 330 can be formed in a precise pattern using a laser or other ablation techniques or by mechanical techniques. As shown, the filter 320 may include a retrieval mechanism. The retrieval mechanism may be connected to the proximal end 320b of the filter 320. For instance, the retrieval mechanism may be connected to the proximal end 320b by the body 338. A retrieval mechanism connected to the proximal end 320b would typically be used to retrieve the filter 320 through an access site that is closer to the proximal end 320b than to the distal end 320a. However, as illustrated, the filter 320 may be removed through an access site that is closer to the proximal end 320b than to the distal end 320a using the body 338. In other embodiments, the filter 320 may include a retrieval mechanism connected to the distal end 320a of the filter 320. In further embodiments, the filter 320 may not include a retrieval mechanism.

The size of apertures 330 can vary along the length of filter 320. For example, the apertures 330 may increase or decrease in size as they approach the proximal end 104. The size of the apertures 330 may transition gradually or abruptly in a proximal or distal direction. The shape of the apertures 330 can be circular, rectangular, square, trapezoidal, oval, slit, or other shapes. The edges of the apertures 330 can be mechanically or chemically chamfered, etched, or polished to provide a smooth and/or rounded shoulder to streamline the passage of blood from within the conical shape portion of the filter 320 to outside of the filter 320. The filter 320 may be configured to capture and/or lyse a variety of particles. The aperture 330 can be sized to reduce stagnation and/or recirculation of bodily fluids (i.e. blood) in and/or around the filter 320 while in use.

The filter 320 may be implanted in a body lumen of the patient. The filter 320 may be inserted and/or retrieved through an access site, such as femoral artery, jugular, or radial vein access sites (shown as 154a, 154b, 154c, respectively, in FIG. 1), or other access sites, or through combinations of these sites. For instance, the filter 320 may be inserted through the jugular vein access site 154b and/or radial vein access site 154c and retrieved through the femoral artery access site 154a. The filter 320 may be deployed near a deployment site (shown as 256 in FIG. 2). The deployment site, for example, may include a location within the inferior vena cava.

FIG. 3B illustrates the filter retrieval system 100 shown in FIG. 1 being delivered to a delivery site (shown as 256 in FIG. 2) of another embodiment of a filter 320'. The filter 320' of this other embodiment may be functionally similar to the filter 320 previously described above and shown in FIG. 3A in most respects, wherein certain features will not be described in relation to this embodiment wherein those components may function in the manner as described above and are hereby incorporated into this alternative embodiment described below. Like structures and/or components are given like reference numerals.

Components of the filter retrieval system 100 may be sized and configured to position the proximal end 116b of the capture structure 116 beyond a distal end 320a' of the filter 320' in a pre-capture state. For instance, the filter retrieval system 100 may be sized and configured to be inserted through at least one aperture 330'. As illustrated in FIGS. 3B and 3B', the filter retrieval system 100 is inserted in a pre-capture state through an aperture 330 near the inner surface of the body lumen 121. In other embodiments, the filter retrieval system 100 may be inserted through any appropriate aperture 330'.

FIG. 4A illustrates the filter retrieval system 100 shown in FIG. 1 engaging the embodiment of a filter 320 shown in the in FIG. 3A. The filter 320 of this other embodiment may be functionally similar to the filter 320' previously described above and shown in FIG. 3B in most respects, wherein certain features will not be described in relation to this embodiment wherein those components may function in the manner as described above and are hereby incorporated into this alternative embodiment described below. Like structures and/or components are given like reference numerals.

As shown, the proximal end 116b of the capture structure 116 is positioned beyond the distal end 320a of the filter 320 in a pre-capture state. As illustrated, the filter retrieval system 100 is inserted in a pre-capture state through an aperture 330 near the center of the body lumen 121 and/or filter 320. In other embodiments, the filter retrieval system 100 may be inserted through any appropriate aperture 330.

The retrieval component may be positioned with respect to the filter 320. For example, the elongate retrieval member 124 may move the retrieval structure 118 to engage the filter 320. The retrieval structure 118 may engage a portion of the body 338. As shown, the retrieval structure 118 may engage a portion of the body 338 near the distal end 320a.

FIG. 4B illustrates the filter retrieval system 100 shown in FIG. 1 engaging the other embodiment of a filter 320 shown in FIG. 3B. The filter 320 of this other embodiment may be functionally similar to the filter 320 previously described above and shown in FIG. 3A in most respects, wherein certain features will not be described in relation to this embodiment wherein those components may function in the manner as described above and are hereby incorporated into this alternative embodiment described below. Like structures and/or components are given like reference numerals.

As shown, the proximal end 116b of the capture structure 116 is positioned beyond the distal end 320a' of the filter 320' in a pre-capture state. As illustrated, the filter retrieval system 100 is inserted in a pre-capture state through an aperture 330' near the inner surface of the body lumen 121. In other embodiments, the filter retrieval system 100 may be inserted through any appropriate aperture 330'.

The retrieval component may be positioned with respect to the filter 320'. For example, the elongate retrieval member 124 may move the retrieval structure 118 to engage the filter 320. The retrieval structure 118 may engage a portion of the body 338. As shown, the retrieval structure 118 may engage a portion of the body 338 generally away from the distal end 320a' (illustrated as closer to the proximal end 320b' than the distal end 320a').

FIG. 5 illustrates directing the embodiment of a filter 320 shown in FIG. 3A into the filter retrieval system 100 shown in FIG. 1. The filter 320 of this other embodiment may be functionally similar to the filter 320 previously described above and shown in FIG. 3A in most respects, wherein certain features will not be described in relation to this embodiment wherein those components may function in the manner as described above and are hereby incorporated into this alternative embodiment described below. Like structures and/or components are given like reference numerals.

At least a portion of the filter 120 may be directed into the capture structure 116. As shown, the entire filter 120 may be directed into the capture structure 116. The filter 120 may transition from a deployed state toward a captured state (i.e. collapsed, longitudinally elongated, etc.).

The capture structure 116 is shown transitioned from an expanded state toward a pre-capture state. As discussed above, the capture structure 116 may expand to receive at least a portion of the filter 120. In embodiments where the capture structure 116 may expand to receive at least a portion of the filter 120, the capture structure 116 may reduce in diameter after receiving at least a portion of the filter 120.

After at least a portion of the filter 120 has been directed into the capture structure 116, the filter retrieval system 100 may be removed from the deployment site (shown as 256 in FIG. 2) through an access site, such as a femoral artery access site, a jugular vein access site, a radial vein access site (shown as 254a, 254b, 254c, respectively in FIG. 2), femoral vein, brachial vein, brachial artery, other access sites, or combinations thereof.

The invention is susceptible to various modifications and alternative means, and specific examples thereof have been shown by way of example in the drawings and are herein described in detail. It should be understood, however, that the invention is not to be limited to the particular devices or methods disclosed, but to the contrary, the invention is to cover all modifications, equivalents, and alternatives falling within the scope of the claims.

## Claims

1. A filter retrieval system (100) comprising:
a delivery catheter (102) having a proximal end (104) and a distal end (106) and defining a lumen (108), said delivery catheter (102) including an aperture (126);
a capture structure (116) operatively associated with said delivery catheter (102) near said distal end (106), the capture structure (116) having an interior space bounded by an at least partially closed distal end and an open proximal end; and
an elongate retrieval member (124) slidably disposable within at least a portion of said interior space, said elongate retrieval member (124) including a retrieval structure (118) adapted to releasably engage at least a portion of a filter (320), said retrieval structure (118) extending out from said lumen (108) through said aperture (126), said aperture (126) extending from a distal end of said capture structure (116) beyond a proximal end of said capture structure.

2. The filter retrieval system (100) of claim 1, wherein said capture structure (116) is configured to be expandable from a collapsed configuration toward an expanded configuration.

3. The filter retrieval system (100) of claim 1, wherein said capture structure (116) includes a tapered proximal portion.

4. The filter retrieval system (100) of claim 1, wherein said retrieval member (124) is moveable between said proximal and distal ends, said retrieval structure (118) being operatively associated with said retrieval member (124).

5. The filter retrieval system (100) of claim 1, said aperture (126) is disposed along an outer wall of said filter retrieval system (100) near said distal end (106) and said retrieval structure (118) extends out from said filter retrieval system (100) through said aperture (126).

6. The filter retrieval system (100) of claim 1, wherein said capture structure (116) is formed from a flexible material and configured to be inflatable to expand from a collapsed configuration toward an expanded configuration.

7. The filter retrieval system (100) of claim 6, wherein said capture structure (116) includes at least one balloon chamber.

8. The filter retrieval system (100) of claim 1, wherein said capture catheter is formed from a flexible material with an elastic member disposed at said proximal end of said capture catheter.

9. The filter retrieval system of claim 1, wherein said retrieval structure (118) is a hook.

10. The filter system of claim 1, wherein said capture structure (116) includes one or more frame members pivotally coupled to a distal end of said capture structure (116).

11. The filter system of claim 10, wherein said capture structure (116) includes an actuator configured to open and close said capture structure (116) by moving said one or more frame members.

## Patentansprüche

1. Filterrücknahmesystem (100), umfassend:
einen Einführkatheder (102), der ein proximales Ende (104) und ein distales Ende (106) hat und ein Lumen (108) definiert, wobei der Einführkatheder (102) eine Öffnung (126) aufweist;
eine Fangstruktur (116), die nahe dem distalen Ende (106) operativ mit dem Einführkatheder (102) verbunden ist, wobei die Fangstruktur (116) einen Innenraum hat, der durch ein mindestens teilweise geschlossenes distales Ende und ein offenes proximales End begrenzt ist; und
ein längliches Rücknahmeglied (124), das verschiebbar innerhalb mindestens eines Bereichs des Innenraums angeordnet ist, wobei das längliche Rücknahmeglied (124) eine Rücknahmestruktur (118), welche geeignet ist, wenigstens einen Teil eines Filters (320) lösbar zu kontaktieren, aufweist, die sich durch die Öffnung (126) hindurch aus dem Lumen (108) heraus erstreckt, wobei die Öffnung (126) sich von einem distalen Ende der Fangstruktur (116) über ein proximales Ende der Fangstruktur hinaus erstreckt.

2. Filterrücknahmesystem (100) nach Anspruch 1, wobei die Fangstruktur (116) dazu gestaltet ist, expandierbar zu sein von einer zusammengefallenen Konfiguration zu einer expandierten Konfiguration.

3. Filterrücknahmesystem (100) nach Anspruch 1, wobei die Fangstruktur (116) einen sich verjüngenden proximalen Bereich aufweist.

4. Filterrücknahmesystem (100) nach Anspruch 1, wobei das Rücknahmeglied (124) zwischen dem proximalen Ende und dem distalen Ende bewegbar ist, wobei die Rücknahmestruktur (118) mit dem Rücknahmeglied (124) operativ verbunden ist.

5. Filterrücknahmesystem (100) nach Anspruch 1, wobei die Öffnung (126) entlang einer Außenwand des Filterrücknahmesystems (100) nahe dem distalen Ende (106) angeordnet ist und sich die Rücknahmestruktur (118) durch die Öffnung (126) hindurch aus dem Filterrücknahmesystem (100) heraus erstreckt.

6. Filterrücknahmesystem (100) nach Anspruch 1, wobei die Fangstruktur (116) aus einem flexiblen Material gebildet ist und dazu gestaltet ist, aufblasbar zu sein, um von einer zusammengefallenen Konfiguration zu einer expandierten Konfiguration zu expandieren.

7. Filterrücknahmesystem (100) nach Anspruch 6, wobei die Fangstruktur (116) mindestens eine Ballonkammer aufweist.

8. Filterrücknahmesystem (100) nach Anspruch 1, wobei der Fangkatheder aus einem flexiblen Material mit einem elastischen Teil gebildet ist, das an dem proximalen Ende des Fangkatheders angeordnet ist.

9. Filterrücknahmesystem nach Anspruch 1, wobei die Rücknahmestruktur (118) ein Haken ist.

10. Filterrücknahmesystem nach Anspruch 1, wobei die Fangstruktur (116) eines oder mehrere Rahmenelemente aufweist, die schwenkbar mit einem distalen Ende der Fangstruktur (116) gekoppelt sind.

11. Filterrücknahmesystem nach Anspruch 10, wobei die Fangstruktur (116) einen Aktuator aufweist, der dazu gestaltet ist, die Fangstruktur (116) durch Bewegen des einen oder der mehreren Rahmenelement zu Öffnen und zu Schließen.

## Revendications

1. Système de récupération de filtre (100) comprenant :
un cathéter de pose (102) ayant une extrémité proximale (104) et une extrémité distale (106) et définissant une lumière (108), ledit cathéter de pose (102) comportant une ouverture (126) ;
une structure de capture (116) associée fonctionnellement audit cathéter de pose (102) près de ladite extrémité distale (106), la structure de capture (116) comportant un espace intérieur délimité par une extrémité distale au moins partiellement fermée et une extrémité proximale ouverte ; et
un élément de récupération allongé (124) qui peut être agencé de façon coulissante à l'intérieur d'au moins une portion dudit espace intérieur, ledit élément de récupération allongé (124) comprenant une structure de récupération (118) adaptée pour s'engager de façon amovible avec au moins une portion d'un filtre (320), ladite structure de récupération (118) s'étendant hors de ladite lumière (108) à travers ladite ouverture (126), ladite ouverture (126) s'étendant depuis une extrémité distale de ladite structure de capture (116) au-delà d'une extrémité proximale de ladite structure de capture.

2. Système de récupération de filtre (100) selon la revendication 1, dans lequel ladite structure de capture (116) est configurée pour être extensible depuis une configuration rétractée vers une configuration déployée.

3. Système de récupération de filtre (100) selon la revendication 1, dans lequel ladite structure de capture (116) comprend une portion proximale conique.

4. Système de récupération de filtre (100) selon la revendication 1, dans lequel ledit élément de récupération (124) est déplaçable entre lesdites extrémités proximale et distale, ladite structure de récupération (118) étant fonctionnellement associée audit élément de récupération (124).

5. Système de récupération de filtre (100) selon la revendication 1, dans lequel ladite ouverture (126) est agencée le long d'une paroi externe dudit système de récupération de filtre (100) près de ladite extrémité distale (106) et ladite structure de récupération (118) s'étend hors dudit système de récupération de filtre (100) à travers ladite ouverture (126).

6. Système de récupération de filtre (100) selon la revendication 1, dans lequel ladite structure de capture (116) est constituée à partir d'un matériau flexible et est configurée pour être gonflable de manière à se déployer depuis une configuration rétractée vers une configuration déployée.

7. Système de récupération de filtre (100) selon la revendication 6, dans lequel ladite structure de capture (116) comprend au moins une chambre de ballon.

8. Système de récupération de filtre (100) selon la revendication 1, dans lequel ledit cathéter de capture est formé à partir d'un matériau flexible avec un élément élastique agencé à ladite extrémité proximale dudit cathéter de capture.

9. Système de récupération de filtre selon la revendication 1, dans lequel ladite structure de récupération (118) est un crochet.

10. Système de filtre selon la revendication 1, dans lequel ladite structure de capture (116) comprend un ou plusieurs éléments de bâti couplés de manière pivotante à une extrémité distale de ladite structure de capture (116).

11. Système de filtre selon la revendication 10, dans lequel ladite structure de capture (116) comprend un actionneur configuré pour ouvrir et fermer ladite structure de capture (116) en déplaçant lesdits un ou plusieurs éléments de bâti.
